## Europäisches Patentamt

**European Patent Office**

**Office européen des brevets**

(11) Publication number: **0 085 891**
**B1**

(12) **EUROPEAN PATENT SPECIFICATION**

(45) Date of publication of patent specification: **29.10.86**

(21) Application number: **83100655.6**

(22) Date of filing: **25.01.83**

(51) Int. Cl.⁴: **A 61 K 7/16**

(54) **A composition for the cleaning and hygien of teeth.**

(30) Priority: **26.01.82 IT 1928782**

(43) Date of publication of application:
**17.08.83 Bulletin 83/33**

(45) Publication of the grant of the patent:
**29.10.86 Bulletin 86/44**

(84) Designated Contracting States:
**AT BE CH DE FR GB LI NL SE**

(56) References cited:
**FR-A-2 258 865**
**GB-A- 476 582**
**GB-A-1 413 642**
**GB-A-1 550 139**
**US-A-1 968 858**
**US-A-3 981 826**

**CHEMICAL ABSTRACTS, vol. 74, no. 20, 17th May 1971, page 201, no. 103049e, Columbus, Ohio, USA**
**Journal of the Americal Dental Association, Vol. 95, pages 982-995, 1977**

**The file contains technical information submitted after the application was filed and not included in this specification**

(73) Proprietor: **LABORATORI BALDACCI Spa**
**Via San Michele degli Scalzi, 73**
**I-56100 Pisa (IT)**

(72) Inventor: **Baldacci, Ugo**
**Via Bruno Buozzi, 20/B**
**I-56100 Pisa (IT)**

(74) Representative: **Barz, Peter, Dr. et al**
**Patentanwälte Dr. V. Schmied-Kowarzik Dipl.-Ing. G. Dannenberg Dr. P. Weinhold Dr. D. Gudel Dipl.-Ing. S. Schubert Dr. P. Barz**
**Siegfriedstrasse 8**
**D-8000 München 40 (DE)**

## Description

The present invention relates to a toothpaste based on water-soluble and non-abrasive substances which are suspended in an excipient (1,2,3-propanetriol) which with said substances give a pleasantly flavoured paste.

Today's ideal toothpaste is a powder of sodium bicarbonate containing 1% of sodium chloride and 1% of stannous fluoride. This excellent combination, based on non-irritating substances, results in a salty taste which is not always agreeable. Besides, it is not easily distributed on a toothbrush.

The commercially available toothpastes are formulated with non-water-soluble chemical substances in which the cation is an alkaline-earth metal cation such as $Ca^{++}$ and $Mg^{++}$. Calcium phosphate, calcium carbonate, calcium sulphate, magnesium hydroxide, magnesium phosphate and magnesium carbonate are the most commonly used substances owing to their abrasive capacity in formulating pastes for cleaning teeth. These substances, which are often micronized to improve the paste homogeneity, are dispersed in excipients which are usually aqueous or comprise water and glycerol or water and alcohol.

When the teeth are brushed with a toothpaste containing such dispersed particles, a part of the particles which are loaded with bacterial deposits that were removed during brushing, adhere to the mucous membranes of the mouth and are not removed during rinsing.

In IT—A—1 044 821 a non-abrasive toothpaste is disclosed which consists of a solid mixture of substances which are all water-soluble and which dissolve in water upon use. The resulting toothpaste, provided either as a gel or as a powder or a micronulate, is even active in the presence of small amounts of water with which the toothbrush has been wetted or moistened beforehand. The gel and granules can be used separately. However, powdered or granulated toothpastes, formed either of non-soluble or soluble salts such as sodium bicarbonate, are not used much. Two-component toothpastes, in which granules are to be added to a gel, are even more difficult to apply.

GB—A—1 413 642 relates to a toothpaste in which the cleaning or abrasive components in addition to sodium bicarbonate comprise other water insoluble substances such as micronized silica, alumina, zirconium silicate, calcium carbonate (in an amount of from 5 to 25% by weight). As a vehicle for this composition, conventional toothpaste vehicles are used, such as mixtures of water and 1,2,3-propanetriol.

Other toothpastes containing water-insoluble abrasive substances in the form of gels or pastes are, for example, described in US—A—1 968 858, Chemical Abstracts, 74, 103049e and in the Journal of the American Dental Association, Vol. 95, pages 982 to 985 (1977).

It is an object of the present invention to provide a toothpaste which is free of water-insoluble abrasive materials and which, by only one application in the form of a suspension contained, for example, in a conventional tube made of plastics or aluminium onto a wet or moistened toothbrush, exhibits an effective cleaning action.

Thus, the invention provides a composition for the cleaning and hygiene of the teeth, which comprises a suspension of water-soluble, non-abrasive salts selected from sodium chloride, sodium bicarbonate, sodium citrate, sodium tartrate, sodium salicylate, magnesium chloride, zinc chloride, strontium acetate, strontium 2-pyrrolidone-5-carboxylate, potassium perchlorate, sodium perborate and tribasic ammonium phosphate in 1,2,3-propanetriol as the only suspending medium, said suspension being free of water-insoluble abrasive materials.

The composition according to the present invention contains the soluble and non-abrasive salts, suspended in pure 1,2,3-propanetriol, as recommended by the odontological pharmacopeas. 1,2,3-propanetriol is devoid of any toxicity and has been proposed as a food component in substitution for carbohydrates in 1929 by Ferma et al., and in 1933 by Johanson et al. A 50% solution of 1,2,3-propanetriol, either intravenously injected or orally administered, results in a reduction of cerebral oedema (G. P. Cantore et al, 1965; M. M. L. Campan et al, 1965; M. Virno et al, 1962).

The suspension, upon being distributed onto a moistened toothbrush of suitable size, forms a supersaturated solution in 1,2,3-propanetriol which, on the one hand, exhibits an effective cleaning action and, on the other hand, causes a flow of liquids from the gingival pockets and from the epithelium of the mucous membranes, possibly affected by inflammatory processes, through the oedematous interstices to the hypertonic solution which is present in the oral cavity.

The 1,2,3-propanetriol does not have any effect on the normal mucous membrane, and it is known (M. Amorosa 1978) that the caustic effect of some compounds (e.g. phenol) is lower if they are in a 1,2,3-propanetriol solution.

The salts suspended in 1,2,3-propanetriol are non-abrasive, soluble in water but insoluble in 1,2,3-propanetriol. Since the alcohol and the salts are water-soluble they do not leave any residue on the mucous membranes when the mouth has been rinsed.

Due to these properties the toothpaste of this invention, upon addition of an antiseptic and/or of an anti-inflammatory agent in a suitable concentration, can be used as a collutory which, if kept in the oral cavity for 2 to 3 minutes, can eliminate the germs which have been separated from the dental plaque during the preceding operations.

Preferably, the antiseptic agent is soluble both in water and in 1,2,3-propanetriol.

The components of the composition of the present invention substantially include surfactants, salts, sweeteners and, optionally, antiseptics.

As surfactant agents, all the synthetic cationic or anionic surfactants and the soaps produced by hydrolysis and subsequent salification of fatty acids deriving from the natural fats can be used.

Among the antiseptic agents, compounds such as 8-hydroxy-quinoline 2-pyrrolidone-5-carboxylate, quinosol, benzethonium chloride, benzalkonium chloride, chlorhexidine diacetate, digluconate or di(2-pyrrolidone-5-carboxylate), cetrimide, cetyl pyridinium chloride, formaldehyde and triformol are useful.

As anti-inflammatory agent the use of 1-(p-chlorobenzoyl)-5-methoxy-2-methyl-1*H*-indole-3-acetic acid or acetylsalicylic acid is preferred. As sweeteners all of the synthetic and natural sweeteners without cariogenic capacity are useful.

All of the components mentioned above are devoid of any abrasive activity and, accordingly, no scratching of the enamel of the tooth takes place and since, in addition, all the components are water-soluble, no solid residue remains in the gingival pockets.

The following specific examples of the compositions of the invention are intended to illustrate the invention without, however, limiting it.

### Example 1

| Component | % |
|---|---|
| Stannous fluoride | 0,1 |
| Tribasic ammonium phosphate | 5 |
| Sodium chloride | 5 |
| Sodium bicarbonate | 30 |
| Menthol | 0,1 |
| Spearmint | 0,1—3 |
| 1,2,3,4,5-Pentanepentol | 30 |
| 1,2,3-propanetriol | to 100 |

### Example 2

| Component | % |
|---|---|
| Sodium bicarbonate | 28.40 |
| Sodium lauryl-sulphonate | 2—4 |
| Menthol | 0—1.2 |
| Spearmint essence | 0.1—3 |
| 1,2,3,4,5-Pentanepentol | 30 |
| Chlorhexidine-di-2-pyrroli-done-5-carboxylate) | 0,1—1 |
| 1,2,3-propanetriol | to 100 |

The chlorhexidine di(2-pyrrolidone-5-carboxylate) antiseptic can be substituted by the following compounds:

| | |
|---|---|
| Quinosol | 0.1—0.5 |
| Benzethonium chloride | 0.1—1 |
| Benzalkonium chloride | 0.1—1 |
| Cetrimide | 0.1—1 |
| Cetyl pyridinium chloride | 0.1—2 |
| Formaldehyde | 0.1—2 |
| Triformol | 0.1—2 |

### Example 3

| Component | % |
|---|---|
| Sodium citrate | 30 |
| Anionic surfactant | 2—4 |
| Spearmint essence | 0.1—3 |
| 1,2,3,4,5-Pentanepentol | 35 |
| Benzethonium chloride | 0.1—1 |
| 1,2,3-propanetriol | to 100 |

### Example 4

| Component | % |
|---|---|
| Sodium chloride | 5 |
| Sodium tartrate | 20 |
| Trisodium citrate | 5 |
| Spearmint essence | 0.1—1 |
| Peppermint essence | 0.1—2 |
| Menthol | 0.1—2 |
| 1,2,3,4,5-Pentanepentol | 35 |
| Cationic or anionic surfactant | 2—4 |
| Formaldehyde | 0.1—2 |
| 1,2,3-propanetriol | to 100 |

### Example 5

| Component | % |
|---|---|
| Sodium bicarbonate | 30 |
| Dry extract of camomile | 1 |
| Spearmint essence | 0.1—1 |
| Menthol | 0.1—2 |
| Peppermint essence | 0.1—3 |
| Triformol | 0.1—2 |
| 1,2,3,4,5-Pentanepentol | 30 |
| 1,2,3-propanetriol | to 100 |

The toothpaste composition of the present invention thus has a composition similar to the so-called powdered toothpastes but in the form of an easy-applicable and flavoured paste having the following properties:

a) Upon distribution onto the toothbrush wetted with water it gives a supersaturated solution which will improve the removal of extraneous substances from the dental surface;

b) Due to hypertonicity of the paste it causes a flow of fluids from the gingival pockets and from any lesion of the mucuous membrane towards the oral cavity;

c) It does not cause any decalcificating and abrasive effect on the dentine;

d) It does not affect the pH value of the oral cavity and it does not inhibit the salivary secretion and the ferments contained therein;

e) It is completely soluble and leaves no residue on the mucous membrane and in the gingival pockets.

A particular aspect of the composition according to the invention is that the use of this composition, as compared with known toothpastes, affords a better separation of dental plaque without leaving traces of any scraping-off or damage to the surface of the teeth.

The toothpaste according to the invention provides considerable improvements in oral hygiene since:

— the excipient and the substances suspended therein are water soluble and, accordingly, do not leave any suspended non-soluble particles adhering to the gums and to the other mucous membranes of the oral cavity;
— is it non-abrasive;
— it has an anti-inflammatory and anti-odemigenic effect;
— it contains antiseptic substances which, upon dissolution in the maximum amount of water which can be retained in the oral cavity, have a sufficient concentration to reduce the cariogenic bacterial flora within one or two minutes;
— it can act as a toothpaste and as a collutory to be applied according to the dentist's prescription.

## Claims

1. A composition for the cleaning and hygiene of the teeth, which comprises a suspension of water-soluble, non-abrasive salts selected from sodium chloride, sodium bicarbonate, sodium citrate, sodium tartrate, sodium salicylate, magnesium chloride, zinc chloride, strontium acetate, strontium 2-pyrrolidone-5-carboxylate, potassium perchlorate, sodium perborate and tribasic ammonium phosphate in 1,2,3-propanetriol as the only suspending medium, said suspension being free of water-insoluble abrasive materials.

2. A composition according to claim 1 which, additionally, comprises an antiseptic agent.

3. A composition according to claim 2 wherein said antiseptic agent is selected from 8-hydroxyquinoline 2-pyrrolidone-5-carboxylate, quinosol, benzethonium chloride, benzalkonium chloride, chlorhexidine diacetate, digluconate or di(2-pyrrolidone-5-carboxylate), cetrimide, cetyl pyridinium chloride, formaldehyde and triformol.

## Patentansprüche

1. Mittel zum Reinigen und zur Hygiene der Zähne, welches umfaßt eine Suspension von wasserlöslichen, nichtabrasiven Salzen, ausgewählt unter Natriumchlorid, Natriumcarbonat, Natriumcitrat, Natriumtartrat, Natriumsalicylat, Magnesiumchlorid, Zinkchlorid, Strontiumacetat, Strontium-2-pyrrolidon-5-carboxylat, Kaliumperchlorat, Natriumperborat und dreibasischem Ammoniumphosphat, in 1,2,3-Propantriol als einzigem Suspendiermedium, wobei die Suspension frei ist von wasserunlöslichen abrasiven Materialien.

2. Mittel nach Anspruch 1, welches zusätzlich ein antiseptisches Mittel umfaßt.

3. Mittel nach Anspruch 2, worin das antiseptische Mittel ausgewählt ist unter 8-Hydrochinolin-2-pyrrolidon-5-carboxylat, Chinosol, Benzethoniumchlorid, Benzalkoniumchlorid, Chlorhexidindiacetat, -digluconat oder -di - (2 - pyrrolidon - 5 - carboxylat), Cetrimid, Cetylpyridiniumchlorid, Formaldehyd und Triformol.

## Revendications

1. Une composition, pour le nettoyage et l'hygiène des dents, qui comprend une suspension de sels non abrasifs, solubles dans l'eau, sélectionnés parmi les substances suivantes: le chlorure de sodium, le bicarbonate de sodium, le citrate de sodium, le tartrate de sodium, le salicylate de sodium, le chlorure de magnésium, le chlorure de zinc, l'acétate de strontium, le pyrrolidone-2-carboxylate-5 de strontium, le perchlorate de potassium, le perborate de sodium et le phosphate d'ammonium tribasique, mis en suspension dans du triol-1,2,3-propane comme seul milieu de suspension, la dite suspension étant dépourvue de toute matière abrasive insoluble dans l'eau.

2. Une composition selon la revendication 1 comprenant, un plus, un agent antiseptique.

3. Une composition selon la revendication 2, dans laquelle ledit agent antiseptique est sélectionné parmi: le pyrrolidone-2 carboxylate-5 d'hydroxy-8 quinoléine, le quinosol, le chlorure de benzethonium, le chlorure de benzalkonium, le diacétate, le digluconate ou le di-(pyrrolidone-2-carboxylate-5) de chlorhexidine, le cétrimide, le chlorure de cétyle pyridinium, le formaldéhyde et le triformol.